(19) Europäisches Patentamt
European Patent Office
Office européen des brevets

(11) EP 4 644 562 A1

(12) EUROPEAN PATENT APPLICATION
published in accordance with Art. 153(4) EPC

(43) Date of publication:
05.11.2025 Bulletin 2025/45

(51) International Patent Classification (IPC):
C12Q 1/68 (2018.01)

(21) Application number: 22969501.0

(52) Cooperative Patent Classification (CPC):
C12Q 1/68

(22) Date of filing: 26.12.2022

(86) International application number:
PCT/CN2022/142103

(87) International publication number:
WO 2024/138344 (04.07.2024 Gazette 2024/27)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(71) Applicant: Qingdao MGI Tech Co.,Ltd
Shandong, 266000 (CN)

(72) Inventors:
• LIU, Guang
Qingdao, Shandong 266000 (CN)
• XU, Guangzhe
Qingdao, Shandong 266000 (CN)
• SHI, Xing
Qingdao, Shandong 266000 (CN)
• CHEN, Wang
Qingdao, Shandong 266000 (CN)

(74) Representative: Body, Marine et al
Papyrus IP SAS
19 rue Ninau
31000 Toulouse (FR)

(54) **GENE SEQUENCING METHOD AND SYSTEM, ELECTRONIC DEVICE, AND STORAGE MEDIUM**

(57) The present disclosure provides a gene sequencing method and system, an electronic device, and a storage medium. The gene sequencing method comprises: acquiring a plurality of groups of signals to be sequenced of a plurality of nucleotide sequence clusters, wherein said plurality of groups of signals have one-to-one correspondence to said plurality of nucleotide sequence clusters, each group of signals to be sequenced comprises a plurality of signals to be sequenced, and each signal to be sequenced corresponds to one sequencing cycle; in each sequencing cycle, obtaining a signal strength value of a corresponding signal to be sequenced; and according to the signal intensity value of each signal to be sequenced, performing identification to obtain a gene sequencing result of the nucleotide sequence cluster corresponding to the corresponding group of signals to be sequenced. According to the present disclosure, independent analysis is performed only on signals to be sequenced to identify a corresponding base sequence, the sequencing operation process is simple and efficient, the requirements for computing resources are greatly reduced, the efficiency and precision in obtaining a gene sequencing result are effectively improved, the error rate is greatly reduced, and the yield and the mapping rate are improved.

FIG. 1

**Description**

TECHNICAL FIELD

The present disclosure relates to the field of gene sequencing technology, and particularly to a gene sequencing method and system, an electronic device, and a storage medium.

BACKGROUND

Existing gene sequencing schemes generally identify each sequencing cycle one by one. After reading all signal data from one sequencing cycle on the sequencing chip, multiple processes such as pre-classification, normalization, and clustering are performed in sequence. After all sequencing cycles are processed, bases at the corresponding positions are concatenated to obtain a final base sequence. As a result, there are problems such as low base identification accuracy and high demand for computational resources in gene sequencing.

SUMMARY

The technical problem to be solved by the present disclosure is to overcome the defects in the prior art, such as low base identification accuracy and high demand for computational resources in gene sequencing, aiming to provide a gene sequencing method and system, an electronic device, and a storage medium.
The present disclosure solves the above technical problems through the following technical solutions:
In a first aspect of the present disclosure, a gene sequencing method is provided, the gene sequencing method comprising:

acquiring a plurality of groups of signals to be sequenced of a plurality of nucleotide sequence clusters;
wherein the plurality of groups of signals to be sequenced have one-to-one correspondence to the plurality of nucleotide sequence clusters, each of the groups of signals to be sequenced comprises a plurality of signals to be sequenced, and each of the signals to be sequenced corresponds to one sequencing cycle;
obtaining a signal intensity value corresponding to the signal to be sequenced in each of the sequencing cycles, respectively;
performing identification to obtain a gene sequencing result of the nucleotide sequence cluster corresponding to corresponding said group of signals to be sequenced according to the signal intensity value of each of the signals to be sequenced.

Preferably, the step of obtaining a signal intensity value corresponding to the signal to be sequenced, respectively, comprises:
obtaining the signal intensity values corresponding to the signals to be sequenced in different preset sequencing channels, respectively; or, obtaining the signal intensity values corresponding to the signals to be sequenced in different preset sequencing time periods, respectively.
Preferably, the step of performing identification to obtain a gene sequencing result of the nucleotide sequence cluster corresponding to corresponding said group of signals to be sequenced according to the signal intensity value of each of the signals to be sequenced comprises:

determining a number of corresponding signal spatial distribution points according to the signal intensity value of each of the signals to be sequenced;
analyzing the signal spatial distribution points using a preset processing rule to obtain a target analysis result;
obtaining the gene sequencing result of the nucleotide sequence cluster corresponding to corresponding said group of signals to be sequenced based on the target analysis result.

Preferably, the step of determining a number of corresponding signal spatial distribution points according to the signal intensity value of each of the signals to be sequenced comprises:

constructing a preset two-dimensional space;
determining spatial position information corresponding to the signal intensity value in the preset two-dimensional space to obtain a number of the signal spatial distribution points distributed in the preset two-dimensional space.

Preferably, the step of analyzing the signal spatial distribution points using a preset processing rule to obtain a target analysis result comprises:

calculating a Euclidean distance between each of the signal spatial distribution points and a preset reference point in the preset two-dimensional space;

performing statistical analysis using a first processing rule according to different said Euclidean distances to obtain a first analysis result;

performing statistical analysis based on the first analysis result using a second processing rule to obtain a second analysis result;

wherein the first analysis result corresponds to identification results of a part of groups, the second analysis result corresponds to identification results of other remaining part of groups, and the target analysis result comprises the first analysis result and the second analysis result.

Preferably, the step of performing statistical analysis using a first processing rule according to different said Euclidean distances to obtain a first analysis result comprises:

generating corresponding first statistical content according to the different said Euclidean distances;

wherein the first statistical content comprises a preset statistical graph, a preset statistical table, or a preset statistical text;

analyzing the first statistical content using the first processing rule to obtain the first analysis result.

Preferably, the step of performing statistical analysis based on the first analysis result using a second processing rule to obtain a second analysis result comprises:

determining an identified group based on the first analysis result;

calculating an arm angle value between the center of the identified group and centers of other groups to be identified;

generating corresponding second statistical content according to different said arm angle values;

wherein the second statistical content comprises a preset statistical graph, a preset statistical table, or a preset statistical text;

analyzing the second statistical content using the second processing rule to obtain the second analysis result.

Preferably, when the first statistical content comprises a first statistical histogram, the step of analyzing the first statistical content using the first processing rule to obtain the first analysis result comprises:

selecting a first longest zero sequence that satisfies a first screening condition in the first statistical histogram;

wherein the first longest zero sequence is a sequence with a longest horizontal length and all vertical values being zero;

obtaining first starting position information of the first longest zero sequence;

determining radius information of a first preset group according to the first starting position information;

identifying a signal spatial distribution point with the Euclidean distance less than the radius information as the first preset group, and other remaining signal spatial distribution points as other groups to be identified.

Preferably, when the second statistical content comprises a second statistical histogram, the step of analyzing the second statistical content using the second processing rule to obtain the second analysis result comprises:

selecting a second longest zero sequence and a sub-longest zero sequence that satisfy a second screening condition in the second statistical histogram;

wherein the second longest zero sequence is a sequence with a longest horizontal length and all vertical values being zero, and the sub-longest zero sequence is a sequence with a sub-longest horizontal length and all vertical values being zero;

distinguishing and identifying the other groups to be identified, respectively, based on the second longest zero sequence, the sub-longest zero sequence, and the arm angle value.

Preferably, the first preset group comprises G group, and the other groups to be identified comprise A group, T group, and C group.

Preferably, before the step of selecting a first longest zero sequence that satisfies a first screening condition in the first statistical histogram, the method comprises:

obtaining a first quantity of histograms with vertical values being zero and arranged consecutively in the first statistical histogram;

determining that current said signal to be sequenced is a bad point, filtering out current said signal to be sequenced, and proceeding to sequencing cycle of next said signal to be sequenced, if the first quantity is less than a set threshold.

Preferably, the step of selecting a first longest zero sequence that satisfies a first screening condition in the first statistical histogram comprises:

determining that current said signal to be sequenced is a good point, and obtaining the first longest zero sequence according to initial positions and end positions corresponding to the first quantity of histograms, if the first quantity is greater than or equal to the set threshold.

Preferably, the gene sequencing method further comprises:

labeling the signal to be sequenced belonging to a good point using preset label information;

storing the signal to be sequenced labeled with the preset label information into a preset storage space of a gene sequencing chip after processing all the signals to be sequenced in the plurality of groups of signals to be sequenced.

Preferably, the gene sequencing method is implemented using multi-core parallel processing hardware.

In a second aspect of the present disclosure, a gene sequencing system is provided, the gene sequencing system comprising:

a to-be-sequenced signal group acquisition module, configured to acquire a plurality of groups of signals to be sequenced of a plurality of nucleotide sequence clusters;

wherein the plurality of groups of signals to be sequenced have one-to-one correspondence to the plurality of nucleotide sequence clusters, each of the groups of signals to be sequenced comprises a plurality of signals to be sequenced, and each of the signals to be sequenced corresponds to one sequencing cycle;

a signal intensity value obtaining module, configured to obtain a signal intensity value corresponding to the signal to be sequenced in each of the sequencing cycles, respectively;

a gene sequencing result obtaining module, configured to perform identification to obtain a gene sequencing result of the nucleotide sequence cluster corresponding to corresponding said group of signals to be sequenced according to the signal intensity value of each of the signals to be sequenced.

Preferably, the signal intensity value obtaining module is configured to obtain the signal intensity values corresponding to the signals to be sequenced in different preset sequencing channels, respectively; or, obtain the signal intensity values corresponding to the signals to be sequenced in different preset sequencing time periods, respectively.

Preferably, the gene sequencing result obtaining module comprises:

a spatial distribution point determination unit, configured to determine a number of corresponding signal spatial distribution points according to the signal intensity value of each of the signals to be sequenced;

a target analysis result obtaining unit, configured to analyze the signal spatial distribution points using a preset processing rule to obtain a target analysis result;

a gene sequencing result obtaining unit, configured to obtain the gene sequencing result of the nucleotide sequence cluster corresponding to corresponding said group of signals to be sequenced based on the target analysis result.

Preferably, the spatial distribution point determination unit comprises:

a two-dimensional space construction subunit, configured to construct a preset two-dimensional space;

a distribution point determination subunit, configured to determine spatial position information corresponding to the signal intensity value in the preset two-dimensional space to obtain a number of the signal spatial distribution points distributed in the preset two-dimensional space.

Preferably, the target analysis result obtaining unit comprises:

a distance calculation subunit, configured to calculate a Euclidean distance between each of the signal spatial distribution points and a preset reference point in the preset two-dimensional space;

a first result obtaining subunit, configured to perform statistical analysis using a first processing rule according to different said Euclidean distances to obtain a first analysis result;

a second result obtaining subunit, configured to perform statistical analysis based on the first analysis result using a second processing rule to obtain a second analysis result;

a target result obtaining subunit, configured to take the first analysis result and the second analysis result as the target analysis result;

wherein the first analysis result corresponds to identification results of a part of groups, and the second analysis result corresponds to identification results of other remaining part of groups.

Preferably, the first result obtaining subunit is further configured to:

generate corresponding first statistical content according to the different said Euclidean distances;
wherein the first statistical content comprises a preset statistical graph, a preset statistical table, or a preset statistical text;
analyze the first statistical content using the first processing rule to obtain the first analysis result.

Preferably, the second result obtaining subunit is further configured to:

determine an identified group based on the first analysis result;
calculate an arm angle value between the center of the identified group and centers of other groups to be identified;
generate corresponding second statistical content according to different said arm angle values;
wherein the second statistical content comprises a preset statistical graph, a preset statistical table, or a preset statistical text;
analyze the second statistical content using the second processing rule to obtain the second analysis result.

Preferably, the first result obtaining subunit is further configured to:

select a first longest zero sequence that satisfies a first screening condition in a first statistical histogram when the first statistical content comprises the first statistical histogram;
wherein the first longest zero sequence is a sequence with a longest horizontal length and all vertical values being zero;
obtain first starting position information of the first longest zero sequence;
determine radius information of a first preset group according to the first starting position information;
identify a signal spatial distribution point with the Euclidean distance less than the radius information as the first preset group, and other remaining signal spatial distribution points as other groups to be identified.

Preferably, the second result obtaining subunit is further configured to:

select a second longest zero sequence and a sub-longest zero sequence that satisfy a second screening condition in a second statistical histogram when the second statistical content comprises the second statistical histogram;
wherein the second longest zero sequence is a sequence with a longest horizontal length and all vertical values being zero, and the sub-longest zero sequence is a sequence with a sub-longest horizontal length and all vertical values being zero;
distinguish and identify the other groups to be identified, respectively, based on the second longest zero sequence, the sub-longest zero sequence, and the arm angle value.

Preferably, the first preset group comprises G group, and the other groups to be identified comprise A group, T group, and C group.
Preferably, the gene sequencing system further comprises:

a first quantity obtaining module, configured to obtain a first quantity of histograms with vertical values being zero and arranged consecutively in the first statistical histogram;
a judgment module, configured to determine that current said signal to be sequenced is a bad point, filter out current said signal to be sequenced, and proceed to sequencing cycle of next said signal to be sequenced, if the first quantity is less than a set threshold.

Preferably, the judgment module is further configured to determine that current said signal to be sequenced is a good point, and invoke the first result obtaining subunit to obtain the first longest zero sequence according to initial positions and end positions corresponding to the first quantity of histograms, if the first quantity is greater than or equal to the set threshold.
Preferably, the gene sequencing system further comprises:

an information labeling module, configured to label the signal to be sequenced belonging to a good point using preset label information;
a signal storage module, configured to store the signal to be sequenced labeled with the preset label information into a preset storage space of a gene sequencing chip after processing all the signals to be sequenced in the plurality of groups of signals to be sequenced.

Preferably, the gene sequencing system further comprises a processor, wherein the processor integrates multi-core parallel processing hardware.

In a third aspect of the present disclosure, a gene sequencing chip is provided, wherein the gene sequencing chip comprises the above gene sequencing system.

In a fourth aspect of the present disclosure, an electronic device is provided, comprising a memory, a processor, and a computer program stored in the memory and executable on the processor, wherein the computer program, when executed by the processor, implements the above gene sequencing method.

In a fifth aspect of the present disclosure, a computer-readable storage medium having a computer program stored thereon is provided, wherein the computer program, when executed by a processor, implements the above gene sequencing method.

On the basis of conforming to common knowledge in the field, the aforementioned preferred conditions may be combined arbitrarily to obtain the preferred examples of the present disclosure.

The positive and progressive effects of the present disclosure lie in that:
In the present disclosure, the group of signals to be sequenced of each nucleotide sequence cluster is acquired, and the signal intensity values of the signals to be sequenced in each of the groups of signals to be sequenced under different preset sequencing conditions at corresponding sequencing cycles are obtained respectively, so as to automatically identify and obtain the gene sequencing results of the nucleotide sequence cluster corresponding to corresponding said group of signals to be sequenced, without considering the influence of signal intensity variations between different groups of signals to be sequenced, without being affected by the number of reads, and without performing quality value filtering separately. Only the signals to be sequenced need to be independently analyzed to identify and obtain the corresponding base sequence. The sequencing operation process is simple and efficient, which significantly reduces the demands for computational resources, effectively improves the efficiency and accuracy of obtaining gene sequencing results, and substantially lowers the error rate, thereby enhancing yield and mapping rate.

BRIEF DESCRIPTION OF THE DRAWINGS

FIG. 1 is a flowchart of a gene sequencing method according to Example 1 of the present disclosure.
FIG. 2 is a first flowchart of a gene sequencing method according to Example 2 of the present disclosure.
FIG. 3 is a second flowchart of the gene sequencing method according to Example 2 of the present disclosure.
FIG. 4 is a third flowchart of the gene sequencing method according to Example 2 of the present disclosure.
FIG. 5 is a schematic diagram of a statistical histogram formed based on Euclidean distance values according to Example 2 of the present disclosure.
FIG. 6 is a schematic diagram of a statistical histogram formed based on arm angle values according to Example 2 of the present disclosure.
FIG. 7 is a schematic diagram of the gene sequencing results according to Example 2 of the present disclosure.
FIG. 8 is an exemplary flowchart of the gene sequencing method according to Example 2 of the present disclosure.
FIG. 9 is a schematic module diagram of a gene sequencing system according to Example 3 of the present disclosure.
FIG. 10 is a schematic module diagram of a gene sequencing system according to Example 4 of the present disclosure.
FIG. 11 is a schematic structural diagram of an electronic device according to Example 5 of the present disclosure.

DETAILED DESCRIPTION OF THE PREFERRED EMBODIMENT

The present disclosure is further illustrated below by way of examples, but the present disclosure is not thereby limited to the scope of the examples.

Gene sequencing refers to the analysis of the base sequence of a specific DNA fragment, namely the arrangement of adenine (A), thymine (T), cytosine (C), and guanine (G). The core principle of second-generation sequencing is sequencing by synthesis, which primarily includes library construction, generation of monoclonal DNA clusters, and sequencing reactions, enabling simultaneous analysis of DNA samples on an array. The reaction carrier for loading DNA is a chip, where DNA undergoes reactions. By measuring the fluorescent groups connected during the reaction, the base sequence at that position is determined. The DNA fluorescence signal on the chip can be acquired through the optical system and scientific camera and converted into a digital signal.

The core principle of second-generation sequencing is sequencing by synthesis. In each biochemical reaction, one base is synthesized, and the sequencer needs to acquire a fluorescence signal from the chip once. Such a process is called a cycle. The result is that in each cycle, one base on the DNA sequence on the chip array can be measured. After several cycles, the DNA sequence on the chip array can be obtained.

In existing gene sequencing schemes, the bases are generally identified according to sequencing cycles one by one. After reading all signal data from one sequencing cycle on the sequencing chip, multiple processes such as pre-classification, normalization, and clustering are performed in sequence. After all sequencing cycles are processed, bases at the corresponding positions are concatenated to obtain a final base sequence. This gene sequencing scheme has the following defects: (1) When there is a significant disparity in signal intensities, interference between bases occurs, leading to substantial deviations in base identification and resulting in low base identification accuracy; (2) The inability to identify whether signals are normal or abnormal prevents the separation of normal and abnormal signals, thereby reducing the final base identification accuracy; (3) Base identification becomes impossible when the number of reads (sequencing fragments) is small, meaning it is affected by the quantity of reads; (4) The overall execution process is complex and demands high computational resources.

**Example 1**

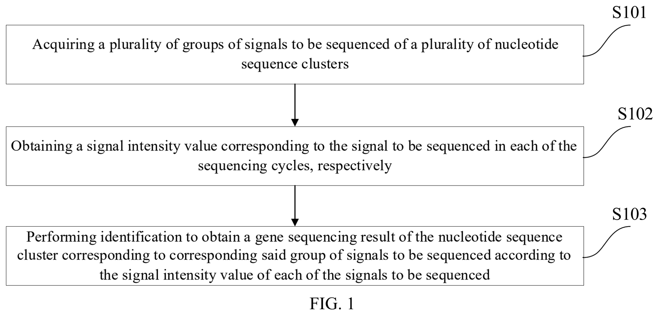

As shown in FIG. 1, a gene sequencing method of this example comprises:

S101: Acquiring a plurality of groups of signals to be sequenced of a plurality of nucleotide sequence clusters; wherein the plurality of groups of signals to be sequenced have one-to-one correspondence to the plurality of nucleotide sequence clusters, that is, each nucleotide sequence cluster corresponds to one group of signals to be sequenced, each of the groups of signals to be sequenced comprises a plurality of signals to be sequenced, and each of the signals to be sequenced corresponds to one sequencing cycle;

Specifically, a cluster is a population of similar or identical molecules or nucleotide sequences or DNA strands; for example, a cluster may be amplified oligonucleotides or any other group of polynucleotides or polypeptides with identical or similar sequences. In other embodiments, a cluster may be any element or group of elements occupying a physical region on the sample surface. In an embodiment, during a base sequencing cycle, clusters are immobilized to reaction sites and/or reaction chambers.

S102: Obtaining a signal intensity value corresponding to the signal to be sequenced in each of the sequencing cycles, respectively;

In each of the sequencing cycles, the signal intensity of the corresponding signal to be sequenced is analyzed under different preset sequencing conditions, respectively, that is, each of the signals to be sequenced undergoes independent analysis and processing. Instead of the current method of identifying different signals to be sequenced according to the sequencing cycles one by one, this approach fundamentally avoids the influence of the mixed processing (such as varying signal intensities, normal and abnormal signals) on gene sequencing results, significantly enhancing the accuracy of gene sequencing results and the processing efficiency of the gene sequencing process.

S103: Performing identification to obtain a gene sequencing result of the nucleotide sequence cluster corresponding to corresponding said group of signals to be sequenced according to the signal intensity value of each of the signals to be sequenced.

In this example, the group of signals to be sequenced of each nucleotide sequence cluster is acquired, and the signal intensity values of the signals to be sequenced in each of the groups of signals to be sequenced under different preset sequencing conditions at corresponding sequencing cycles are obtained respectively, so as to automatically identify and obtain the gene sequencing results of the nucleotide sequence cluster corresponding to corresponding said group of signals to be sequenced, without considering the influence of signal intensity variations between different groups of signals to be sequenced, without being affected by the number of reads, and without performing quality value filtering separately. Only the signals to be sequenced need to be independently analyzed to identify and obtain the corresponding base sequence. The sequencing operation process is simple and efficient, which significantly reduces the demands for computational resources, effectively improves the efficiency and accuracy of obtaining gene sequencing results, and substantially lowers the error rate, thereby enhancing yield and mapping rate.

**Example 2**

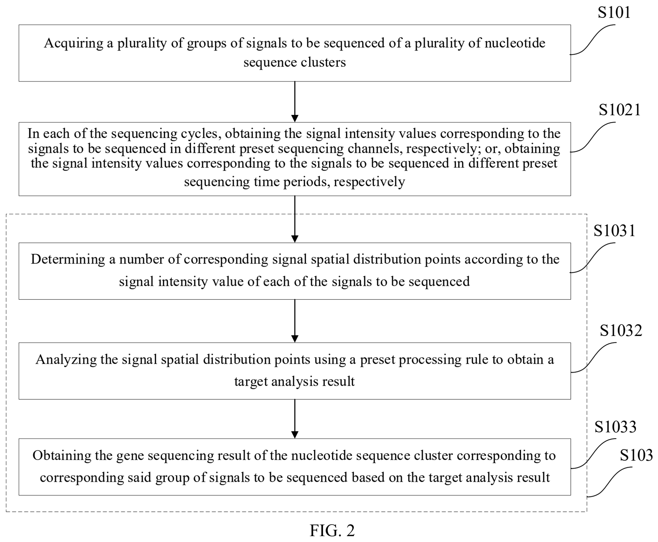

The gene sequencing method of this example is a further improvement of Example 1, specifically:
In an implementable embodiment, as shown in FIG. 2, step S102 comprises:
S1021: In each of the sequencing cycles, obtaining the signal intensity values corresponding to the signals to be sequenced in different preset sequencing channels, respectively; or, obtaining the signal intensity values corresponding to the signals to be sequenced in different preset sequencing time periods, respectively.

It is certain that, in addition to the preset sequencing conditions of different preset sequencing channels and different preset sequencing time periods, there may be other sequencing conditions capable of independently analyzing the signal to be sequenced, which will not be described in detail here.

In an implementable embodiment, step S103 comprises:

S1031: Determining a number of corresponding signal spatial distribution points according to the signal intensity value of each of the signals to be sequenced;

S1032: Analyzing the signal spatial distribution points using a preset processing rule to obtain a target analysis result;

S1033: Obtaining the gene sequencing result of the nucleotide sequence cluster corresponding to corresponding said group of signals to be sequenced based on the target analysis result.

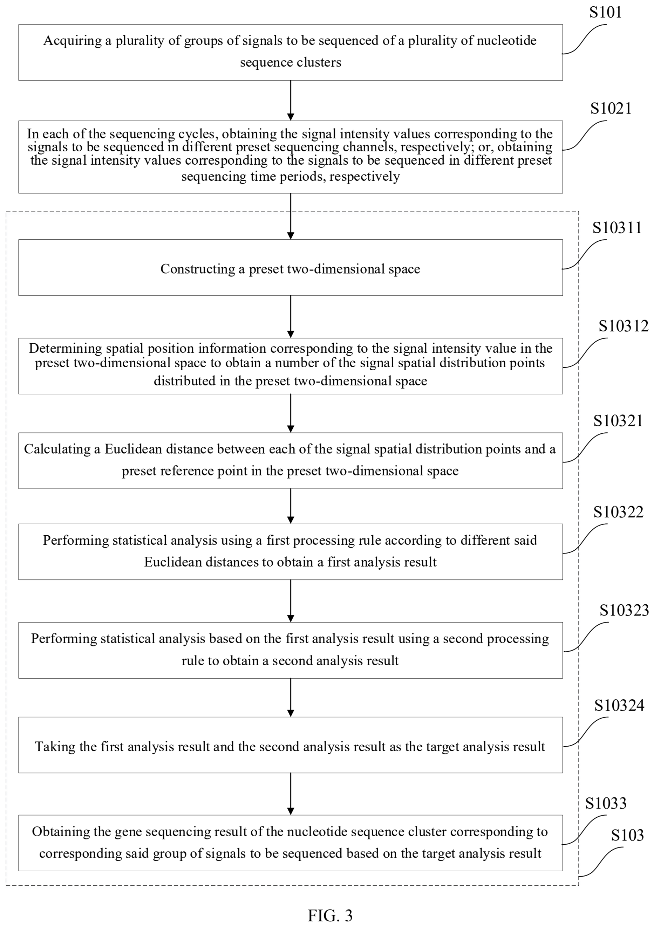

In an implementable embodiment, as shown in FIG. 3, step S1031 comprises:

S10311: Constructing a preset two-dimensional space;

S10312: Determining spatial position information corresponding to the signal intensity value in the preset two-dimensional space to obtain a number of the signal spatial distribution points distributed in the preset two-dimensional space.

Specifically, when the different preset sequencing channels correspond to a first preset sequencing channel and a second preset sequencing channel, the signal intensity values correspond to a first signal intensity in the first preset sequencing channel and a second signal intensity in the second preset sequencing channel.

The constructed preset two-dimensional space is a two-dimensional coordinate system, with the first preset sequencing channel as the abscissa and the second preset sequencing channel as the ordinate, obtaining a number of the signal spatial distribution points within the two-dimensional coordinate system.

In an implementable embodiment, step S1032 comprises:

S10321: Calculating a Euclidean distance between each of the signal spatial distribution points and a preset reference point in the preset two-dimensional space;

wherein the Euclidean distance between each of the signal spatial distribution points and the coordinate origin of the two-dimensional coordinate system is calculated; the method for calculating the Euclidean distance between any two spatial coordinate points is a mature technology in the field and thus will not be repeated here.

S10322: Performing statistical analysis using a first processing rule according to different said Euclidean distances to obtain a first analysis result;

wherein the first processing rule includes but is not limited to: a rule for finding the longest zero sequence based on a Euclidean distance statistical table.

S10323: Performing statistical analysis based on the first analysis result using a second processing rule to obtain a second analysis result;

wherein the first processing rule includes but is not limited to: a rule for finding the longest zero sequence based on an angle statistical table.

S10324: Taking the first analysis result and the second analysis result as the target analysis result;

wherein the first analysis result corresponds to identification results of a part of groups, and the second analysis result corresponds to identification results of other remaining part of groups.

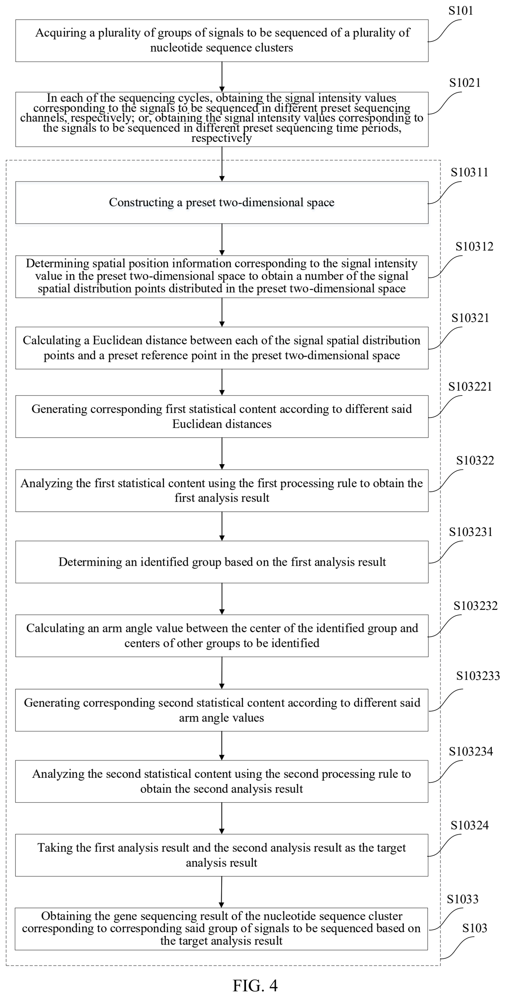

In an implementable embodiment, as shown in FIG. 4, step S10322 comprises:

S103221: Generating corresponding first statistical content according to different said Euclidean distances;

wherein the first statistical content comprises a preset statistical graph, a preset statistical table, or a preset statistical text, etc.;

S103222: Analyzing the first statistical content using the first processing rule to obtain the first analysis result.

In an implementable embodiment, step S10323 comprises:

S103231: Determining an identified group based on the first analysis result;

S103232: Calculating an arm angle value between the center of the identified group and centers of other groups to be identified;

S103233: Generating corresponding second statistical content according to different said arm angle values;

wherein the second statistical content comprises a preset statistical graph, a preset statistical table, or a preset statistical text, etc.;

S103234: Analyzing the second statistical content using the second processing rule to obtain the second analysis result.

In an implementable embodiment, the first statistical content comprises a first statistical histogram, and the number of the first statistical histograms equals the number of sequencing cycles. Step S103222 comprises:

Selecting a first longest zero sequence that satisfies a first screening condition in the first statistical histogram; wherein the first longest zero sequence is a sequence with a longest horizontal length and all vertical values being zero;

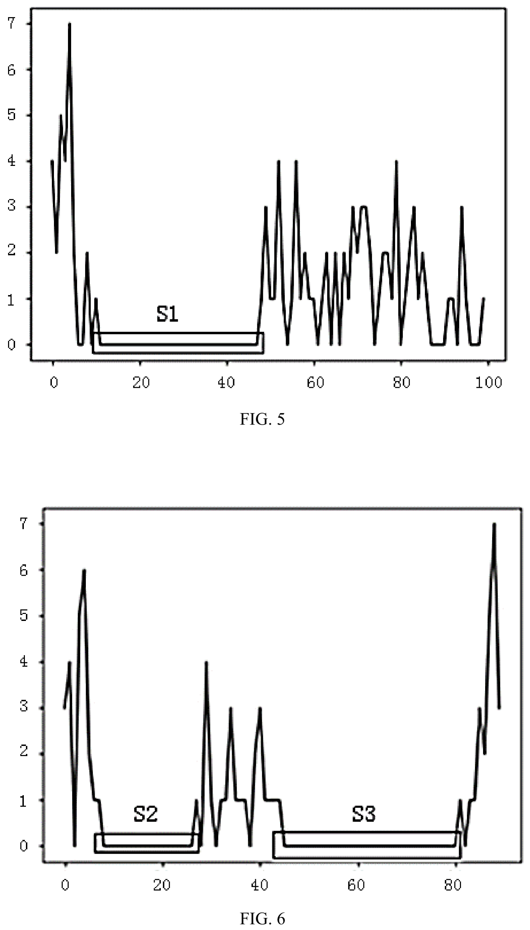

Specifically, as shown in FIG. 5, which illustrates the first statistical histogram obtained based on the statistics of all Euclidean distance values, the first longest zero sequence to be found is shown in the black box S1 in the figure.

Obtaining first starting position information of the first longest zero sequence;

Determining radius information of a first preset group according to the first starting position information; Identifying a signal spatial distribution point with the Euclidean distance less than the radius information as the first preset group, and other remaining signal spatial distribution points as other groups to be identified.

Herein, the first preset group comprises G group, and the other groups to be identified comprise A group, T group, and C group.

Based on the first statistical histogram, the G base group is identified by finding the first longest zero sequence, the radius and center of the G base group are determined, and further the identification results of other ACT bases are obtained, thereby ensuring the accuracy of base identification.

Specifically, before the step of selecting a first longest zero sequence that satisfies a first screening condition in the first statistical histogram, the method comprises:

obtaining a first quantity of histograms with vertical values being zero and arranged consecutively in the first statistical histogram; determining that current said signal to be sequenced is a bad point, filtering out current said signal to be sequenced, and proceeding to sequencing cycle of next said signal to be sequenced, if the first quantity is less than a set threshold.

In this embodiment, all abnormal signals to be sequenced that fail to satisfy the conditions are filtered out, retaining only normal signals to be sequenced for determining the final gene sequencing results, thereby further ensuring the accuracy and reliability of the sequencing results.

Herein, the step of selecting a first longest zero sequence that satisfies a first screening condition in the first statistical histogram comprises:
determining that current said signal to be sequenced is a good point, and obtaining the first longest zero sequence according to initial positions and end positions corresponding to the first quantity of histograms, if the first quantity is greater than or equal to the set threshold.

In this embodiment, the conventional method of filtering signal bad points using Q values is abandoned, and the length of the first longest zero sequence is used for filtering, which improves the accuracy of bad point identification and simplifies the filtering process, thereby effectively improving the accuracy and efficiency of the final gene sequencing.

In an implementable embodiment, when the second statistical content comprises a second statistical histogram, step S103234 comprises:

selecting a second longest zero sequence and a sub-longest zero sequence that satisfy a second screening condition in the second statistical histogram; wherein the second longest zero sequence is a sequence with a longest horizontal length and all vertical values being zero, and the sub-longest zero sequence is a sequence with a sub-longest horizontal length and all vertical values being zero; Specifically, as shown in FIG. 6, corresponding to the second statistical histogram obtained based on the statistics of all arm angle values, the second longest zero sequence and the sub-longest zero sequence to be found are shown in the black boxes S2 and S3 in the figure, respectively.

The other groups to be identified are distinguished and identified, respectively, based on the second longest zero sequence, the sub-longest zero sequence, and the arm angle value.

In this embodiment, two target arm angle values between the center of the first preset group under the second longest zero sequence and the sub-longest zero sequence and any two signal spatial distribution points in other preset groups are respectively calculated; based on the two target arm angle values, the group type corresponding to each other preset group is determined.

Specifically, among the two target arm angle values, if one target arm angle value is greater than a preset angle and the other target arm angle value is less than the preset angle, both target arm angle values are determined to be valid, and based on the two target arm angle values, the group type corresponding to each other preset group is determined.

Otherwise, third-longest zero sequences corresponding to several second statistical histograms are obtained; two new target arm angle values between the center of the first preset group under the first longest zero sequence and the third-longest zero sequence and any two signal spatial distribution points in other preset groups are respectively calculated; based on the two new target arm angle values, the group type corresponding to each other preset group is determined.

Herein, after determining that the arm angle value is valid, the step of determining the group type corresponding to each other group based on the two target arm angle values comprises:

selecting a signal spatial distribution point, from the signal spatial distribution points, where the arm angle value to the center of the first preset group is greater than the larger of the two target arm angle values, thereby forming an adenine A group;

selecting a signal spatial distribution point, from the signal spatial distribution points, where the arm angle value to the center of the first preset group is smaller than the smaller of the two target arm angle values, thereby forming a cytosine T group;

forming a thymine C group based on the remaining signal spatial distribution points.

In an implementable embodiment, the gene sequencing method further comprises:

labeling the signal to be sequenced belonging to a good point using preset label information;

storing the signal to be sequenced labeled with the preset label information into a preset storage space of a gene sequencing chip after processing all the signals to be sequenced in the plurality of groups of signals to be sequenced.

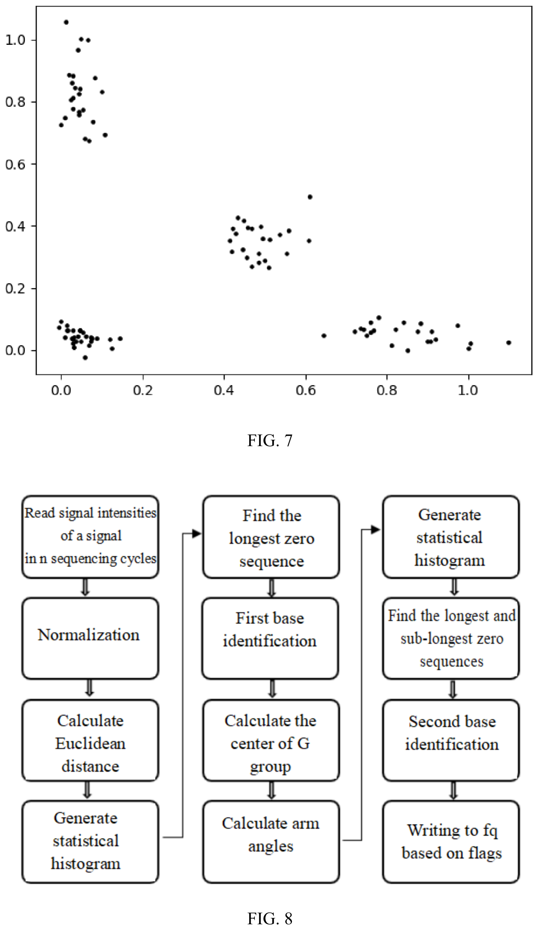

As shown in FIG. 7, the distribution plot of signal intensity after normalization, obtained by reading the signal intensity values corresponding to the signals to be sequenced in different preset sequencing channels for one sequencing cycle based on the gene sequencing method of this example, can demonstrate a good clustering phenomenon, thereby ensuring the accuracy of gene sequencing results.

In an implementable embodiment, the gene sequencing method is implemented using multi-core parallel processing hardware.

Specifically, compared with existing gene sequencing schemes, the gene sequencing process in this example demands fewer computational resources and is more convenient for use in practical products, thereby reducing costs. If multi-core parallel processing hardware such as GPU (graphics processing unit) and FPGA (field programmable gate array) is used to accelerate computing, the efficiency will be significantly better than existing gene sequencing schemes. The prerequisite for parallel computing is that a task can be divided into several unrelated subtasks. In the context of this method, each DNB (a nanoball sequencing approach based on patterned arrays for in situ sequencing) can be treated as an independent computational subtask. Additionally, the advantage of GPUs over CPUs is that GPUs have hundreds to thousands of times more processing cores than CPUs. Each processing core can execute tasks in parallel, allowing all DNB data processing to be assigned to the processing cores of GPU for parallel execution, thereby achieving higher processing speeds compared to CPUs.

The gene sequencing method of this example can be applied to sequencing platforms such as NGS (high-throughput sequencing technology) second-generation sequencing platform.

The implementation principle of the gene sequencing method of this example is illustrated in detail below with reference to an instance (see FIG. 8):

(1) Acquiring a plurality of groups of signals to be sequenced of a plurality of nucleotide sequence clusters; wherein the plurality of groups of signals to be sequenced have one-to-one correspondence to the plurality of nucleotide sequence clusters, each of the groups of signals to be sequenced comprises n signals to be sequenced (where n is an integer), and each of the signals to be sequenced corresponds to one sequencing cycle;

(2) Obtaining, in each of the sequencing cycles, a first signal intensity signal1 of the corresponding signal to be sequenced in a first preset sequencing channel image2 and a second signal intensity signal2 in a second preset sequencing channel image2, respectively;

(3) Constructing a two-dimensional coordinate system, using the first preset sequencing channel image1 as the x-coordinate and the second preset sequencing channel image2 as the y-coordinate, thereby obtaining a number of corresponding signal spatial distribution points; wherein the x-coordinate of each of the signal spatial distribution points corresponds to the first signal intensity signal1, and the y-coordinate corresponds to the second signal intensity signal2;

(4) Normalizing the signal intensity and coordinate values to ensure the feasibility of subsequent data calculations and simplify the complexity of data processing; calculating the Euclidean distance (distance) between each of the signal spatial distribution points and the coordinate origin of the two-dimensional coordinate system based on the normalized values, with the Euclidean distance formula as follows:

$$distance = \sqrt{signal1^2 + signal2^2}$$

(5) Statistically processing a number of Euclidean distances obtained in step (4) (taking the distance values between 1% and 99%), so as to obtain a first statistical histogram;

wherein the number of histograms (num_hist) in the first statistical histogram is the same as the number of sequencing cycles, e.g., selecting a first statistical histogram containing 100 histograms for 100 sequencing cycles.

Here, excluding distance values below 1% and above 99% is equivalent to filtering out potential interference values, ensuring the accuracy of the subsequent acquisition of the first longest zero sequence, thereby further enhancing the precision of the final gene sequencing results;

(6) Selecting a first longest zero sequence that satisfies a first screening condition in the first statistical histogram; wherein the first longest zero sequence is a sequence with a longest horizontal length and all vertical values being zero; the detailed steps are as follows:

1) Sequentially determining whether the vertical value of each histogram is 0; if the vertical value of the i-th histogram is 0, incrementing number by one; if the vertical value of the i-th histogram is not 0, saving the signal spatial distribution point i at the current position to the array location, saving number to the array location_number, and simultaneously resetting number to zero;

2) Extracting a maximum value max_value from the array location_number, and simultaneously obtaining a maximum value subscript max_subscript; if max_value is less than a set value (e.g., 7), then setting the flag corresponding to the signal spatial distribution point to 1, indicating that the signal to be sequenced is a bad point and needs be discarded, thereby directly filtering out abnormal signals; if max_value is greater than or equal to 7, then setting the flag corresponding to the signal spatial distribution point to 0, indicating that the signal to be sequenced is a good point, and obtaining the values with the subscripts max_subscript-1 and max_subscript from the array location_number, using the value at max_subscript-1 as the initial position x1 of the first longest zero sequence and the value at max_subscript as the end position x2 of the first longest zero sequence;

(7) Calculating the radius RadiusG of the G base group based on the initial position x1 and the end position x2 of the first longest zero sequence obtained in step (6), with the corresponding formula as follows:

$$RadiusG = \frac{x1 + x2}{2} \times \frac{P99 - P1}{num\_hist}$$

wherein P1 refers to the value at the 1% position among all distance values obtained in step (4), and P99 refers to the value at the 99% position among all distance values obtained in step (4);

(8) Identifying the G base group (i.e., the first preset group) based on the distance values obtained in step (4) wherein signal spatial distribution points with the Euclidean distances in step (4) less than RadiusG are identified as G, while other remaining signal spatial distribution points are temporarily identified as other group types, i.e., ACT groups (i.e., other groups to be identified);

(9) Calculating the center of the G base group

Based on the identification results in step (8), the signal intensity values and numbers of all groups identified as G base group in the first preset sequencing channel image1 and the second preset sequencing channel image2 are calculated, with the corresponding formula as follows:

$$centerGx = \frac{\sum_0^m signal1[i]}{m}$$

$$centerGy = \frac{\sum_0^p signal2[i]}{m}$$

wherein m represents the number of G bases identified in any channel, and the number of G bases identified in the first preset sequencing channel image1 is equal to that in the second preset sequencing channel image2; centerGx and centerGy represent the x-coordinate and y-coordinate of the center of the G base group, respectively; the number of G base groups in different preset sequencing channels is directly obtained by counting the identification results in step (8), which will not be repeated here.

(10) Calculating the clustering angles of other ACT groups to be identified

Based on the center of the G base group obtained in step (9), calculating the arm angle value slop between the signal spatial distribution points identified as ACT groups in step (8) and the center of the G base group, with the corresponding arm angle value slop calculated as follows:

$$slop = arctan\left(\frac{signal2 - centerGy}{signal1 - centerGx}\right) \times \frac{180}{\pi}$$

(11) Counting the arm angle values slop (taking arm angle values between 1% and 99%) obtained in step (10) to obtain a second statistical histogram, e.g., the number of the second statistical histograms is 90;

Here, excluding arm angle values below 1% and above 99% is equivalent to filtering out potential interference values, ensuring the accuracy of the subsequent second longest zero sequence and sub-longest zero sequence, thereby further enhancing the precision of the final gene sequencing results.

(12) Determining the second longest zero sequence and the sub-longest zero sequence in the second statistical histogram using the same method as in step (6), with the implementation process being identical, which will not be repeated here;

(13) Combining step (10) to calculate two corresponding arm angle values, slop1 and slop2, for the second longest zero sequence and the sub-longest zero sequence, respectively;

(14) Obtaining the ACT boundary angles slopAC and slopTC, with the corresponding calculation formulas as follows:

$$slopAC = max\{slop1, slop2\}$$

$$slopTC = min\{slop1, slop2\}$$

(15) Based on the identification results of the G base group in step (8), re-identifying the remaining signal spatial distribution points corresponding to other groups to be identified, specifically:

identifying the signal spatial distribution points with arm angle values in step (11) greater than slopAC as A group, identifying the signal spatial distribution points with arm angle values in step (11) less than slopTC as T group, and identifying the remaining signal spatial distribution points as C group;

It should be noted that the arm angle values slop1 and slop2 need to satisfy the condition where one is greater than 45 degrees and the other is less than 45 degrees; otherwise, it indicates that the current arm angle values are deemed invalid, requiring the search for a third-longest zero sequence in the second statistical histogram; the third-longest zero sequence is used to replace the sub-longest zero sequence, and steps (12)-(15) are repeated until new arm angle values slop1 and slop2 are calculated to satisfy the condition of one being greater than 45 degrees and the other being less than 45 degrees; if the newly obtained zero sequences consistently fail to satisfy the condition where one of the arm angle values slop1 and slop2 is greater than 45 degrees and the other is less than 45 degrees, it indicates that valid sequencing results cannot be obtained, and the gene sequencing operation is terminated;

(16) Returning to step (1), reading the next signal to be sequenced, and iteratively executing all the above steps until all signals to be sequenced in the group of signals to be sequenced corresponding to each nucleotide sequence cluster are processed;

(17) After all signals to be sequenced are processed, when writing to fq, if the flag corresponding to the signal to be sequenced in step (6) is 0, that is, the signal to be sequenced is a good point representing a normal signal, performing fq writing operation to store the signal into a preset storage space of the gene sequencing chip; if the flag corresponding to the signal to be sequenced is 1, that is, the signal to be sequenced is a bad point representing an abnormal signal, not writing to fq, thereby achieving the purpose of automatically filtering abnormal signals.

In addition, in order to test the effect of the gene sequencing scheme in this example, sequencing output data from two gene sequencing chips of an NGS platform were randomly selected. The data for gene sequencing chips No. 1 and No. 2 were randomly extracted from rectangular regions of size $817 \times 765$ on each respective chip, while the data for chip No. 3 corresponded to the full-chip sequencing output data of chip No. 2. The test results are shown in the table below:

| Chip No. | Total (M) | Number of Mappings (M) | Mapping rate (%) | Error rate (%) | Non-N error rate (%) |
| --- | --- | --- | --- | --- | --- |
| 1 | 0.419 | 0.418 | 99.64 | 0.12 | 0.11 |
| 2 | 0.418 | 0.416 | 99.69 | 0.12 | 0.12 |
| 3 | 5.811 | 5.791 | 99.67 | 0.14 | 0.13 |

After testing, it is found that the gene sequencing scheme in this example achieves a mapping rate above 99.6% and an error rate below 0.15%, whether testing the entire chip or randomly selected regions on the chip. The scheme also demonstrates consistency across different chips and exhibits high robustness. Therefore, it can be shown that the gene sequencing scheme in this example is feasible and capable of completing a gene sequencing operation with high quality.

In this example, the group of signals to be sequenced of each nucleotide sequence cluster is acquired, and the signal intensity values of the signals to be sequenced in each of the groups of signals to be sequenced under different preset sequencing conditions at corresponding sequencing cycles are obtained respectively, so as to automatically identify and obtain the gene sequencing results of the nucleotide sequence cluster corresponding to corresponding said group of signals to be sequenced, without considering the influence of signal intensity variations between different groups of signals to be sequenced, without being affected by the number of reads, and without performing quality value filtering separately. Only the signals to be sequenced need to be independently analyzed to identify and obtain the corresponding base sequence. The sequencing operation process is simple and efficient, which significantly reduces the demands for computational resources, effectively improves the efficiency and accuracy of obtaining gene sequencing results, and substantially lowers the error rate, thereby enhancing yield and mapping rate.

## Example 3

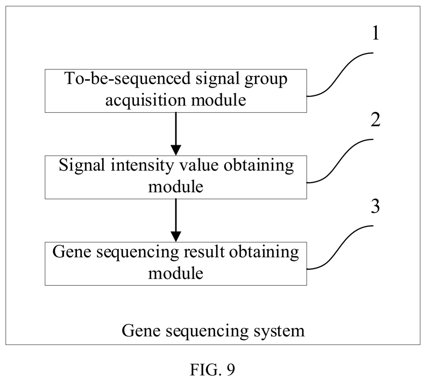

As shown in FIG. 9, a gene sequencing system of this example comprises:

a to-be-sequenced signal group acquisition module 1, configured to acquire a plurality of groups of signals to be sequenced of a plurality of nucleotide sequence clusters;
wherein the plurality of groups of signals to be sequenced have one-to-one correspondence to the plurality of nucleotide sequence clusters, that is, each nucleotide sequence cluster corresponds to one group of signals to be sequenced, each of the groups of signals to be sequenced comprises a plurality of signals to be sequenced, and each of the signals to be sequenced corresponds to one sequencing cycle;
Specifically, a cluster is a population of similar or identical molecules or nucleotide sequences or DNA strands; for example, a cluster may be amplified oligonucleotides or any other group of polynucleotides or polypeptides with identical or similar sequences. In other embodiments, a cluster may be any element or group of elements occupying a physical region on the sample surface. In an embodiment, during a base sequencing cycle, clusters are immobilized to reaction sites and/or reaction chambers.

A signal intensity value obtaining module 2, configured to obtain a signal intensity value corresponding to the signal to be sequenced in each of the sequencing cycles, respectively;
In each of the sequencing cycles, the signal intensity of the corresponding signal to be sequenced is analyzed under different preset sequencing conditions, respectively, that is, each of the signals to be sequenced undergoes independent analysis and processing. Instead of the current method of identifying different signals to be sequenced according to the sequencing cycles one by one, this approach fundamentally avoids the influence of the mixed processing (such as varying signal intensities, normal and abnormal signals) on gene sequencing results, significantly enhancing the accuracy of gene sequencing results and the processing efficiency of the gene sequencing process.

A gene sequencing result obtaining module 3, configured to perform identification to obtain a gene sequencing result of the nucleotide sequence cluster corresponding to corresponding said group of signals to be sequenced according to the signal intensity value of each of the signals to be sequenced.

It should be noted that the implementation principle of the gene sequencing system in this example is the same as that of the gene sequencing method in Example 1, and thus will not be repeated here.

In this example, the group of signals to be sequenced of each nucleotide sequence cluster is acquired, and the signal intensity values of the signals to be sequenced in each of the groups of signals to be sequenced under different preset sequencing conditions at corresponding sequencing cycles are obtained respectively, so as to automatically identify and obtain the gene sequencing results of the nucleotide sequence cluster corresponding to corresponding said group of signals to be sequenced, without considering the influence of signal intensity variations between different groups of signals to be sequenced, without being affected by the number of reads, and without performing quality value filtering separately. Only the signals to be sequenced need to be independently analyzed to identify and obtain the corresponding base sequence. The sequencing operation process is simple and efficient, which significantly reduces the demands for computational resources, effectively improves the efficiency and accuracy of obtaining gene sequencing results, and substantially lowers the error rate, thereby enhancing yield and mapping rate.

**Example 4**

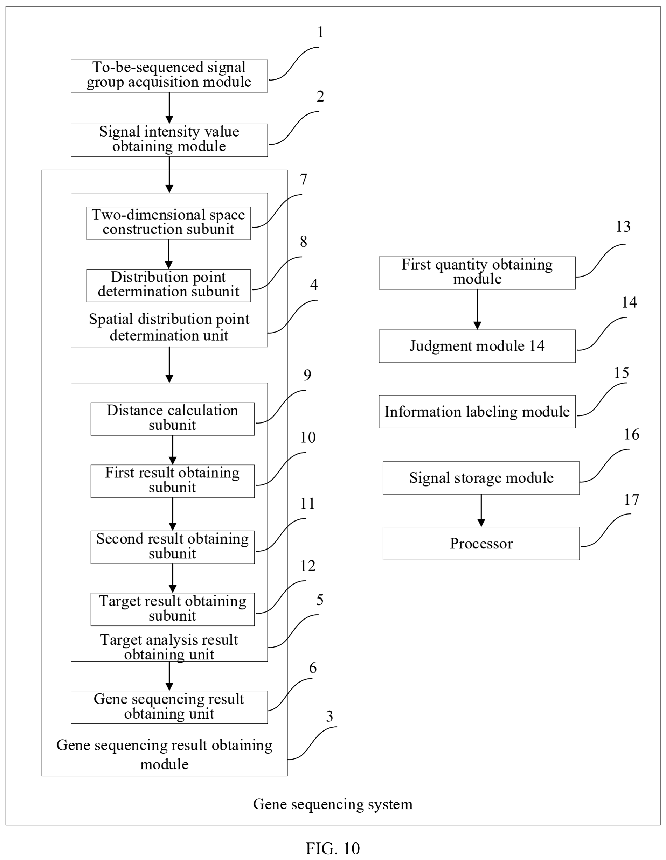

As shown in FIG. 10, the gene sequencing system of this example is a further improvement of Example 3, specifically:

In an implementable embodiment, the signal intensity value obtaining module 2 is configured to obtain, in each of the sequencing cycles, the signal intensity values corresponding to the signals to be sequenced in different preset sequencing channels, respectively; or, obtain the signal intensity values corresponding to the signals to be sequenced in different preset sequencing time periods, respectively.

It is certain that, in addition to the preset sequencing conditions of different preset sequencing channels and different preset sequencing time periods, there may be other sequencing conditions capable of independently analyzing the signal to be sequenced, which will not be described in detail here.

In an implementable embodiment, the gene sequencing result obtaining module 3 comprises:

a spatial distribution point determination unit 4, configured to determine a number of corresponding signal spatial distribution points according to the signal intensity value of each of the signals to be sequenced;
a target analysis result obtaining unit 5, configured to analyze the signal spatial distribution points using a preset processing rule to obtain a target analysis result;
a gene sequencing result obtaining unit 6, configured to obtain the gene sequencing result of the nucleotide sequence cluster corresponding to corresponding said group of signals to be sequenced based on the target analysis result.

In an implementable embodiment, the spatial distribution point determination unit 4 comprises:

a two-dimensional space construction subunit 7, configured to construct a preset two-dimensional space;
a distribution point determination subunit 8, configured to determine spatial position information corresponding to the signal intensity value in the preset two-dimensional space to obtain a number of the signal spatial distribution points distributed in the preset two-dimensional space.

Specifically, when the different preset sequencing channels correspond to a first preset sequencing channel and a second preset sequencing channel, the signal intensity values correspond to a first signal intensity in the first preset sequencing channel and a second signal intensity in the second preset sequencing channel.

The constructed preset two-dimensional space is a two-dimensional coordinate system, with the first preset sequencing channel as the abscissa and the second preset sequencing channel as the ordinate, obtaining a number of the signal spatial distribution points within the two-dimensional coordinate system.

In an implementable embodiment, the target analysis result obtaining unit 5 comprises:

a distance calculation subunit 9, configured to calculate a Euclidean distance between each of the signal spatial distribution points and a preset reference point in the preset two-dimensional space;
wherein the Euclidean distance between each of the signal spatial distribution points and the coordinate origin of the two-dimensional coordinate system is calculated; the method for calculating the Euclidean distance between any two spatial coordinate points is a mature technology in the field and thus will not be repeated here;
a first result obtaining subunit 10, configured to perform statistical analysis using a first processing rule according to different said Euclidean distances to obtain a first analysis result;
wherein the first processing rule includes but is not limited to: a rule for finding the longest zero sequence based on a Euclidean distance statistical table;
a second result obtaining subunit 11, configured to perform statistical analysis based on the first analysis result using a second processing rule to obtain a second analysis result;

wherein the first processing rule includes but is not limited to: a rule for finding the longest zero sequence based on an angle statistical table;

a target result obtaining subunit 12, configured to take the first analysis result and the second analysis result as the target analysis result;

wherein the first analysis result corresponds to identification results of a part of groups, and the second analysis result corresponds to identification results of other remaining part of groups.

In an implementable embodiment, the first result obtaining subunit 10 is further configured to:

generate corresponding first statistical content according to different said Euclidean distances;

wherein the first statistical content comprises a preset statistical graph, a preset statistical table, or a preset statistical text;

analyze the first statistical content using the first processing rule to obtain the first analysis result.

In an implementable embodiment, the second result obtaining subunit 11 is further configured to:

determine an identified group based on the first analysis result;

calculate an arm angle value between the center of the identified group and centers of other groups to be identified;

generate corresponding second statistical content according to different said arm angle values;

wherein the second statistical content comprises a preset statistical graph, a preset statistical table, or a preset statistical text;

analyze the second statistical content using the second processing rule to obtain the second analysis result.

In an implementable embodiment, the first statistical content comprises a first statistical histogram, and the number of the first statistical histograms equals the number of sequencing cycles.

The first result obtaining subunit 10 is further configured to:

Select a first longest zero sequence that satisfies a first screening condition in the first statistical histogram;

wherein the first longest zero sequence is a sequence with a longest horizontal length and all vertical values being zero;

Specifically, as shown in FIG. 5, which illustrates the first statistical histogram obtained based on the statistics of all Euclidean distance values, the first longest zero sequence to be found is shown in the black box S1 in the figure.

Obtain first starting position information of the first longest zero sequence;

Determine radius information of a first preset group according to the first starting position information;

Identify a signal spatial distribution point with the Euclidean distance less than the radius information as the first preset group, and other remaining signal spatial distribution points as other groups to be identified.

Herein, the first preset group comprises G group, and the other groups to be identified comprise A group, T group, and C group.

Based on the first statistical histogram, the G base group is identified by finding the first longest zero sequence, the radius and center of the G base group are determined, and further the identification results of other ACT bases are obtained, thereby ensuring the accuracy of base identification.

In an implementable embodiment, the gene sequencing system further comprises:

a first quantity obtaining module 13, configured to obtain a first quantity of histograms with vertical values being zero and arranged consecutively in the first statistical histogram;

a judgment module 14, configured to determine that current said signal to be sequenced is a bad point, filter out current said signal to be sequenced, and proceed to sequencing cycle of next said signal to be sequenced, if the first quantity is less than a set threshold.

In this embodiment, all abnormal signals to be sequenced that fail to satisfy the conditions are filtered out, retaining only normal signals to be sequenced for determining the final gene sequencing results, thereby further ensuring the accuracy and reliability of the sequencing results.

In an implementable embodiment, the judgment module 14 is further configured to determine that current said signal to be sequenced is a good point, and invoke the first result obtaining subunit to obtain the first longest zero sequence according to initial positions and end positions corresponding to the first quantity of histograms, if the first quantity is greater than or equal to the set threshold.

In this embodiment, the conventional method of filtering signal bad points using Q values is abandoned, and the length of the first longest zero sequence is used for filtering, which improves the accuracy of bad point identification and simplifies the filtering process, thereby effectively improving the accuracy and efficiency of the final gene sequencing.

In an implementable embodiment, the second result obtaining subunit 11 is further configured to:

select a second longest zero sequence and a sub-longest zero sequence that satisfy a second screening condition in a second statistical histogram when the second statistical content comprises the second statistical histogram; wherein the second longest zero sequence is a sequence with a longest horizontal length and all vertical values being zero, and the sub-longest zero sequence is a sequence with a sub-longest horizontal length and all vertical values being zero;

Specifically, as shown in FIG. 6, corresponding to the second statistical histogram obtained based on the statistics of all arm angle values, the second longest zero sequence and the sub-longest zero sequence to be found are shown in the black boxes S2 and S3 in the figure, respectively.

The other groups to be identified are distinguished and identified, respectively, based on the second longest zero sequence, the sub-longest zero sequence, and the arm angle value.

In this embodiment, two target arm angle values between the center of the first preset group under the second longest zero sequence and the sub-longest zero sequence and any two signal spatial distribution points in other preset groups are respectively calculated; based on the two target arm angle values, the group type corresponding to each other preset group is determined.

Specifically, among the two target arm angle values, if one target arm angle value is greater than a preset angle and the other target arm angle value is less than the preset angle, both target arm angle values are determined to be valid, and based on the two target arm angle values, the group type corresponding to each other preset group is determined.

Otherwise, third-longest zero sequences corresponding to several second statistical histograms are obtained; two new target arm angle values between the center of the first preset group under the first longest zero sequence and the third-longest zero sequence and any two signal spatial distribution points in other preset groups are respectively calculated; based on the two new target arm angle values, the group type corresponding to each other preset group is determined.

Herein, after determining that the arm angle value is valid, the step of determining the group type corresponding to each other group based on the two target arm angle values comprises:

selecting a signal spatial distribution point, from the signal spatial distribution points, where the arm angle value to the center of the first preset group is greater than the larger of the two target arm angle values, thereby forming an adenine A group; selecting a signal spatial distribution point, from the signal spatial distribution points, where the arm angle value to the center of the first preset group is smaller than the smaller of the two target arm angle values, thereby forming a cytosine T group; forming a thymine C group based on the remaining signal spatial distribution points.

In an implementable embodiment, the gene sequencing system further comprises:

an information labeling module 15, configured to label the signal to be sequenced belonging to a good point using preset label information; a signal storage module 16, configured to store the signal to be sequenced labeled with the preset label information into a preset storage space of a gene sequencing chip after processing all the signals to be sequenced in the plurality of groups of signals to be sequenced.

As shown in FIG. 7, the distribution plot of signal intensity after normalization, obtained by reading the signal intensity values corresponding to the signals to be sequenced in different preset sequencing channels for one sequencing cycle based on the gene sequencing method of this example, can demonstrate a good clustering phenomenon, thereby ensuring the accuracy of gene sequencing results.

In an implementable embodiment, the gene sequencing system further comprises a processor 17, wherein the processor 17 integrates multi-core parallel processing hardware.

Specifically, compared with existing gene sequencing schemes, the gene sequencing process in this example demands fewer computational resources and is more convenient for use in practical products, thereby reducing costs. If multi-core parallel processing hardware such as GPU (graphics processing unit) and FPGA (field programmable gate array) is used to accelerate computing, the efficiency will be significantly better than existing gene sequencing schemes.

The prerequisite for parallel computing is that a task can be divided into several unrelated subtasks. In the context of this method, each DNB can be treated as an independent computational subtask. Additionally, the advantage of GPUs over CPUs is that GPUs have hundreds to thousands of times more processing cores than CPUs. Each processing core can execute tasks in parallel, allowing all DNB data processing to be assigned to the processing cores of GPU for parallel execution, thereby achieving higher processing speeds compared to CPUs.

The gene sequencing method of this example can be applied to sequencing platforms such as NGS second-generation sequencing platform.

It should be noted that the implementation principle of the gene sequencing system in this example is the same as that of the gene sequencing method in Example 2, and thus will not be repeated here.

In this example, the group of signals to be sequenced of each nucleotide sequence cluster is acquired, and the signal intensity values of the signals to be sequenced in each of the groups of signals to be sequenced under different preset sequencing conditions at corresponding sequencing cycles are obtained respectively, so as to automatically identify and obtain the gene sequencing results of the nucleotide sequence cluster corresponding to corresponding said group of signals to be sequenced, without considering the influence of signal intensity variations between different groups of signals to be sequenced, without being affected by the number of reads, and without performing quality value filtering separately. Only the signals to be sequenced need to be independently analyzed to identify and obtain the corresponding base sequence. The sequencing operation process is simple and efficient, which significantly reduces the demands for computational resources, effectively improves the efficiency and accuracy of obtaining gene sequencing results, and substantially lowers the error rate, thereby enhancing yield and mapping rate.

## Example 5

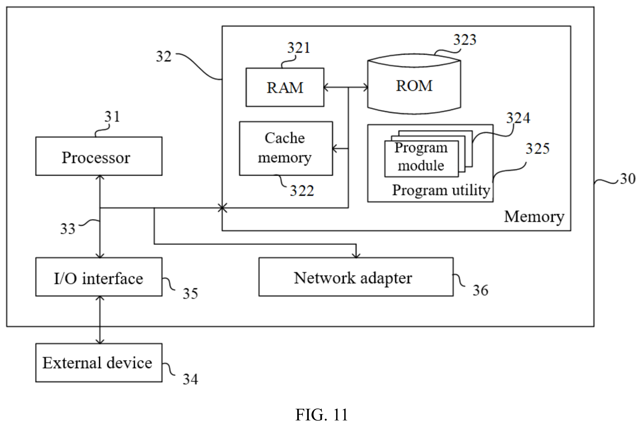

FIG. 11 is a schematic structural diagram of an electronic device provided in Example 5 of the present disclosure. The electronic device comprises a memory, a processor, and a computer program stored in the memory and executable on the processor, wherein the program, when executed by the processor, implements the method in the above examples. The electronic device 30 shown in FIG. 11 is merely an example and should not impose any limitations on the functions and usage scope of the examples of the present disclosure.

As shown in FIG. 11, the electronic device 30 may be in the form of a general-purpose computing device, for example, it may be a server device. The components of the electronic device 30 may include, but are not limited to, at least one processor 31, at least one memory 32, and a bus 33 that connects various system components (including the memory 32 and the processor 31).

The bus 33 comprises a data bus, an address bus, and a control bus.

The memory 32 may comprise a volatile memory, such as a random-access memory (RAM) 321 and/or a cache memory 322, and may further comprise a read-only memory (ROM) 323.

The memory 32 may further comprise a program/utility 325 with a set of (at least one) program modules 324 including, but not limited to, an operating system, one or more application programs, other program modules, and program data. Each of these examples or some combination thereof may comprise a component for implementing a network environment.

The processor 31 executes the computer program stored in the memory 32, thereby executing various functional applications and data processing, such as the method in the above examples of the present disclosure.

The electronic device 30 may also communicate with one or more external devices 34 (*e.g.,* a keyboard or pointing device). Such communication is established through an input/output (I/O) interface 35. Moreover, the model-generated device 30 may also communicate with one or more networks (*e.g.*, a local area network (LAN), a wide area network (WAN), and/or a public network, such as the Internet) via a network adapter 36. As shown in FIG. 11, the network adapter 36 communicates with other modules of the model-generated device 30 via the bus 33. It should be understood that although not shown in figures, other hardware and/or software modules can be used in combination with the model-generated electronic device 30, including, but not limited to, a microcode, a device driver, a redundant processor, an external disk drive array, a RAID (Redundant Array of Independent Disks) system, a tape drive, a data backup storage system, etc.

It should be noted that although several units/modules or sub-units/modules of the electronic device are mentioned in the detailed description above, such a division is merely exemplary and not mandatory. In practice, according to the embodiments of the present disclosure, the features and functions of two or more units/modules described above may be embodied in a single unit/module. Conversely, the features and functions of a single unit/module described above may be further divided to be embodied by multiple units/modules.

## Example 6

This example provides a computer-readable storage medium having a computer program stored thereon, wherein the program, when executed by a processor, implements the steps of the method in the above examples.

Herein, the readable storage medium may more specifically include, but is not limited to, a portable disk, a hard disk, a random-access memory, a read-only memory, an erasable programmable read-only memory, an optical storage device, a magnetic storage device, or any suitable combination of the above.

In a possible embodiment, the present disclosure may also be implemented in the form of a program product comprising a program code which, when the program product is run on a terminal device, causes the terminal device to perform the steps of the method in the above examples.

Herein, the program code for executing the present disclosure may be written in any combination of one or more programming languages, and may be executed entirely on the user device, partially on the user device, as a standalone software package, partially on the user device and partially on a remote device, or entirely on a remote device.

Although specific embodiments of the present disclosure are described above, those skilled in the art should understand that these are merely illustrative examples. A variety of changes or modifications can be made to these embodiments without departing from the principles and essence of the present disclosure. Therefore, the scope of protection of the present disclosure is defined by the appended claims.

**Claims**

1. A gene sequencing method, **characterized in that** the gene sequencing method comprises:

   acquiring a plurality of groups of signals to be sequenced of a plurality of nucleotide sequence clusters;
   wherein the plurality of groups of signals to be sequenced have one-to-one correspondence to the plurality of nucleotide sequence clusters, each of the groups of signals to be sequenced comprises a plurality of signals to be sequenced, and each of the signals to be sequenced corresponds to one sequencing cycle;
   obtaining a signal intensity value corresponding to the signal to be sequenced in each of the sequencing cycles, respectively;
   performing identification to obtain a gene sequencing result of the nucleotide sequence cluster corresponding to corresponding said group of signals to be sequenced according to the signal intensity value of each of the signals to be sequenced.

2. The gene sequencing method according to claim 1, **characterized in that** the step of obtaining a signal intensity value corresponding to the signal to be sequenced respectively comprises:
   obtaining the signal intensity values corresponding to the signals to be sequenced in different preset sequencing channels, respectively; or, obtaining the signal intensity values corresponding to the signals to be sequenced in different preset sequencing time periods, respectively.

3. The gene sequencing method according to claim 1 or 2, **characterized in that** the step of performing identification to obtain a gene sequencing result of the nucleotide sequence cluster corresponding to corresponding said group of signals to be sequenced according to the signal intensity value of each of the signals to be sequenced, comprises:

   determining a number of corresponding signal spatial distribution points according to the signal intensity value of each of the signals to be sequenced;
   analyzing the signal spatial distribution points using a preset processing rule to obtain a target analysis result;
   obtaining the gene sequencing result of the nucleotide sequence cluster corresponding to corresponding said group of signals to be sequenced based on the target analysis result.

4. The gene sequencing method according to at least one of claims 1 to 3, **characterized in that** the step of determining a number of corresponding signal spatial distribution points according to the signal intensity value of each of the signals to be sequenced comprises:

   constructing a preset two-dimensional space;
   determining spatial position information corresponding to the signal intensity value in the preset two-dimensional space to obtain a number of the signal spatial distribution points distributed in the preset two-dimensional space.

5. The gene sequencing method according to claim 4, **characterized in that** the step of analyzing the signal spatial distribution points using a preset processing rule to obtain a target analysis result comprises:

   calculating a Euclidean distance between each of the signal spatial distribution points and a preset reference point in the preset two-dimensional space;

performing statistical analysis using a first processing rule according to different said Euclidean distances to obtain a first analysis result;

performing statistical analysis based on the first analysis result using a second processing rule to obtain a second analysis result;

taking the first analysis result and the second analysis result as the target analysis result;

wherein the first analysis result corresponds to identification results of a part of groups, and the second analysis result corresponds to identification results of other remaining part of groups.

6. The gene sequencing method according to claim 5, **characterized in that** the step of performing statistical analysis using a first processing rule according to different said Euclidean distances to obtain a first analysis result comprises:

generating corresponding first statistical content according to the different said Euclidean distances;

wherein the first statistical content comprises a preset statistical graph, a preset statistical table, or a preset statistical text;

analyzing the first statistical content using the first processing rule to obtain the first analysis result.

7. The gene sequencing method according to claim 5 or 6, **characterized in that** the step of performing statistical analysis based on the first analysis result using a second processing rule to obtain a second analysis result comprises:

determining an identified group based on the first analysis result;

calculating an arm angle value between the center of the identified group and centers of other groups to be identified;

generating corresponding second statistical content according to different said arm angle values;

wherein the second statistical content comprises a preset statistical graph, a preset statistical table, or a preset statistical text;

analyzing the second statistical content using the second processing rule to obtain the second analysis result.

8. The gene sequencing method according to claim 6 or 7, **characterized in that** when the first statistical content comprises a first statistical histogram, the step of analyzing the first statistical content using the first processing rule to obtain the first analysis result comprises:

selecting a first longest zero sequence that satisfies a first screening condition in the first statistical histogram;

wherein the first longest zero sequence is a sequence with a longest horizontal length and all vertical values being zero;

obtaining first starting position information of the first longest zero sequence;

determining radius information of a first preset group according to the first starting position information;

identifying a signal spatial distribution point with the Euclidean distance less than the radius information as the first preset group, and other remaining signal spatial distribution points as other groups to be identified.

9. The gene sequencing method according to claim 7 or 8, **characterized in that** when the second statistical content comprises a second statistical histogram, the step of analyzing the second statistical content using the second processing rule to obtain the second analysis result comprises:

selecting a second longest zero sequence and a sub-longest zero sequence that satisfy a second screening condition in the second statistical histogram;

wherein the second longest zero sequence is a sequence with a longest horizontal length and all vertical values being zero, and the sub-longest zero sequence is a sequence with a sub-longest horizontal length and all vertical values being zero;

distinguishing and identifying the other groups to be identified, respectively, based on the second longest zero sequence, the sub-longest zero sequence, and the arm angle values.

10. The gene sequencing method according to claim 8 or 9, **characterized in that** the first preset group comprises G group, and the other groups to be identified comprise A group, T group, and C group.

11. The gene sequencing method according to at least one of claims 8 to 10, **characterized in that** before the step of selecting a first longest zero sequence that satisfies a first screening condition in the first statistical histogram, the method comprises:

obtaining a first quantity of histograms with vertical values being zero and arranged consecutively in the first statistical histogram;

determining that current said signal to be sequenced is a bad point, filtering out current said signal to be sequenced, and proceeding to sequencing cycle of next said signal to be sequenced, if the first quantity is less than a set threshold.

12. The gene sequencing method according to claim 11, **characterized in that** the step of selecting a first longest zero sequence that satisfies a first screening condition in the first statistical histogram comprises:

determining that current said signal to be sequenced is a good point, and obtaining the first longest zero sequence according to initial positions and end positions corresponding to the first quantity of histograms, if the first quantity is greater than or equal to the set threshold.

13. The gene sequencing method according to claim 12, **characterized in that** the gene sequencing method further comprises:

labeling the signal to be sequenced belonging to a good point using preset label information;

storing the signal to be sequenced labeled with the preset label information into a preset storage space of a gene sequencing chip after processing all the signals to be sequenced in the plurality of groups of signals to be sequenced.

14. The gene sequencing method according to at least one of claims 1 to 13, **characterized in that** the gene sequencing method is implemented using multi-core parallel processing hardware.

15. A gene sequencing system, **characterized in that** the gene sequencing system comprises:

a to-be-sequenced signal group acquisition module, configured to acquire a plurality of groups of signals to be sequenced of a plurality of nucleotide sequence clusters;

wherein the plurality of groups of signals to be sequenced have one-to-one correspondence to the plurality of nucleotide sequence clusters, each of the groups of signals to be sequenced comprises a plurality of signals to be sequenced, and each of the signals to be sequenced corresponds to one sequencing cycle;

a signal intensity value obtaining module, configured to obtain a signal intensity value corresponding to the signal to be sequenced in each of the sequencing cycles, respectively;

a gene sequencing result obtaining module, configured to perform identification to obtain a gene sequencing result of the nucleotide sequence cluster corresponding to corresponding said group of signals to be sequenced according to the signal intensity value of each of the signals to be sequenced.

16. A gene sequencing chip, **characterized in that** the gene sequencing chip comprises the gene sequencing system according to claim 14.

17. An electronic device comprising a memory, a processor, and a computer program stored in the memory and executable on the processor, **characterized in that** the computer program, when executed by the processor, implements the gene sequencing method according to any one of claims 1 to 14.

18. A computer-readable storage medium having a computer program stored thereon, **characterized in that** the computer program, when executed by a processor, implements the gene sequencing method according to any one of claims 1 to 14.

S101

Acquiring a plurality of groups of signals to be sequenced of a plurality of nucleotide sequence clusters

S102

Obtaining a signal intensity value corresponding to the signal to be sequenced in each of the sequencing cycles, respectively

S103

Performing identification to obtain a gene sequencing result of the nucleotide sequence cluster corresponding to corresponding said group of signals to be sequenced according to the signal intensity value of each of the signals to be sequenced

FIG. 1

S101

Acquiring a plurality of groups of signals to be sequenced of a plurality of nucleotide sequence clusters

S1021

In each of the sequencing cycles, obtaining the signal intensity values corresponding to the signals to be sequenced in different preset sequencing channels, respectively; or, obtaining the signal intensity values corresponding to the signals to be sequenced in different preset sequencing time periods, respectively

S1031

Determining a number of corresponding signal spatial distribution points according to the signal intensity value of each of the signals to be sequenced

S1032

Analyzing the signal spatial distribution points using a preset processing rule to obtain a target analysis result

S1033

S103

Obtaining the gene sequencing result of the nucleotide sequence cluster corresponding to corresponding said group of signals to be sequenced based on the target analysis result

FIG. 2

S101

Acquiring a plurality of groups of signals to be sequenced of a plurality of nucleotide sequence clusters

S1021

In each of the sequencing cycles, obtaining the signal intensity values corresponding to the signals to be sequenced in different preset sequencing channels, respectively; or, obtaining the signal intensity values corresponding to the signals to be sequenced in different preset sequencing time periods, respectively

S10311

Constructing a preset two-dimensional space

S10312

Determining spatial position information corresponding to the signal intensity value in the preset two-dimensional space to obtain a number of the signal spatial distribution points distributed in the preset two-dimensional space

S10321

Calculating a Euclidean distance between each of the signal spatial distribution points and a preset reference point in the preset two-dimensional space

S10322

Performing statistical analysis using a first processing rule according to different said Euclidean distances to obtain a first analysis result

S10323

Performing statistical analysis based on the first analysis result using a second processing rule to obtain a second analysis result

S10324

Taking the first analysis result and the second analysis result as the target analysis result

S1033
S103

Obtaining the gene sequencing result of the nucleotide sequence cluster corresponding to corresponding said group of signals to be sequenced based on the target analysis result

FIG. 3

| | |
|---|---|
| Acquiring a plurality of groups of signals to be sequenced of a plurality of nucleotide sequence clusters | S101 |

↓

| | |
|---|---|
| In each of the sequencing cycles, obtaining the signal intensity values corresponding to the signals to be sequenced in different preset sequencing channels, respectively; or, obtaining the signal intensity values corresponding to the signals to be sequenced in different preset sequencing time periods, respectively | S1021 |

↓

| | |
|---|---|
| Constructing a preset two-dimensional space | S10311 |

↓

| | |
|---|---|
| Determining spatial position information corresponding to the signal intensity value in the preset two-dimensional space to obtain a number of the signal spatial distribution points distributed in the preset two-dimensional space | S10312 |

↓

| | |
|---|---|
| Calculating a Euclidean distance between each of the signal spatial distribution points and a preset reference point in the preset two-dimensional space | S10321 |

↓

| | |
|---|---|
| Generating corresponding first statistical content according to different said Euclidean distances | S103221 |

↓

| | |
|---|---|
| Analyzing the first statistical content using the first processing rule to obtain the first analysis result | S10322 |

↓

| | |
|---|---|
| Determining an identified group based on the first analysis result | S103231 |

↓

| | |
|---|---|
| Calculating an arm angle value between the center of the identified group and centers of other groups to be identified | S103232 |

↓

| | |
|---|---|
| Generating corresponding second statistical content according to different said arm angle values | S103233 |

↓

| | |
|---|---|
| Analyzing the second statistical content using the second processing rule to obtain the second analysis result | S103234 |

↓

| | |
|---|---|
| Taking the first analysis result and the second analysis result as the target analysis result | S10324 |

↓

| | |
|---|---|
| Obtaining the gene sequencing result of the nucleotide sequence cluster corresponding to corresponding said group of signals to be sequenced based on the target analysis result | S1033 / S103 |

FIG. 4

FIG. 5

FIG. 6

FIG. 7

FIG. 8

FIG. 9

To-be-sequenced signal group acquisition module — 1

Signal intensity value obtaining module — 2

Two-dimensional space construction subunit — 7

Distribution point determination subunit — 8

Spatial distribution point determination unit — 4

Distance calculation subunit — 9

First result obtaining subunit — 10

Second result obtaining subunit — 11

Target result obtaining subunit — 12

Target analysis result obtaining unit — 5

Gene sequencing result obtaining unit — 6

Gene sequencing result obtaining module — 3

First quantity obtaining module — 13

Judgment module 14 — 14

Information labeling module — 15

Signal storage module — 16

Processor — 17

Gene sequencing system

FIG. 10

FIG. 11

## INTERNATIONAL SEARCH REPORT

| International application No. |
| --- |
| **PCT/CN2022/142103** |

| A. | CLASSIFICATION OF SUBJECT MATTER |
| --- | --- |

C12Q 1/68(2018.01)i

According to International Patent Classification (IPC) or to both national classification and IPC

| B. | FIELDS SEARCHED |
| --- | --- |

Minimum documentation searched (classification system followed by classification symbols)

IPC: C12Q

Documentation searched other than minimum documentation to the extent that such documents are included in the fields searched

Electronic data base consulted during the international search (name of data base and, where practicable, search terms used)

VEN, CNABS, CNTXT, WOTXT, EPTXT, USTXT, CNKI, IEEE: 基因, 测序, 核苷酸, 序列, 信号, 组, 测序循环, 强度, gene, sequence, nucleotide, signal, group, cycle, intensity

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | CN 115376613 A (ZHENGZHOU SIKUN BIOLOGICAL ENGINEERING CO., LTD.) 22 November 2022 (2022-11-22) description, paragraphs 78-149 | 1-18 |
| A | CN 106434856 A (BGI SHENZHEN et al.) 22 February 2017 (2017-02-22) entire document | 1-18 |
| A | CN 110964793 A (SHENZHEN MGI JICHUANG TECHNOLOGY CO., LTD.) 07 April 2020 (2020-04-07) entire document | 1-18 |
| A | US 2019106746 A1 (GEORGETOWN UNIVERSITY) 11 April 2019 (2019-04-11) entire document | 1-18 |

☐ Further documents are listed in the continuation of Box C.    ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **14 June 2023** | **19 June 2023** |

| Name and mailing address of the ISA/CN | Authorized officer |
| --- | --- |
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT
### Information on patent family members

International application No.

**PCT/CN2022/142103**

| Patent document cited in search report | | | Publication date (day/month/year) | Patent family member(s) | | | Publication date (day/month/year) |
|---|---|---|---|---|---|---|---|
| CN | 115376613 | A | 22 November 2022 | None | | | |
| CN | 106434856 | A | 22 February 2017 | None | | | |
| CN | 110964793 | A | 07 April 2020 | None | | | |
| US | 2019106746 | A1 | 11 April 2019 | WO | 2017160686 | A1 | 21 September 2017 |

Form PCT/ISA/210 (patent family annex) (July 2022)